# EUROPEAN PATENT APPLICATION

(11) **EP 4 509 175 A1**
(43) Date of publication of application: **19.02.2025**
(21) Application number: 23788412.7
(22) Date of filing: 14.04.2023
(51) Int. Cl.: A61P 17/16, A61Q 19/00, A61K 9/06, A61K 9/10, A61K 8/06, A61K 8/46, A61K 31/185

(54) **SKIN EXTERNAL AGENT COMPOSITION IN WHICH FINE NEEDLE-LIKE OR COLUMNAR CRYSTALS OF TAURINE ARE BLENDED**

(30) Priority: 15.04.2022 JP 2022067637
(71) Applicant: Cosmed Pharmaceutical Co., Ltd., Kyoto-shi, Kyoto 601-8014 (JP)
(72) Inventor: MURAYAMA, Tomohiro, Kyoto-shi, Kyoto 601-8014 (JP); TANAKA, Hiroshi, Kyoto-shi, Kyoto 601-8014 (JP); HASEGAWA, Junya, Kyoto-shi, Kyoto 601-8014 (JP); QUAN, Ying-shu, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2023/015116
(87) International publication number: WO 2023/199995

(57) **Abstract**

Provided is a skin external agent composition containing taurine as crystals, which has smooth feel without any skin contact. A skin external agent composition containing fine needle-like or columnar crystals of taurine having a thickness of less than 150 micrometers and a length of less than 3000 micrometers, a method for producing fine needle-like or columnar crystals of taurine, the method including a step of growing crystals in an environment in which an aqueous solution in which taurine is dissolved is mixed with or brought into contact with a liquid substance having low solubility of taurine, and a method for producing a skin external agent composition, the method including the step of growing crystals and a step of blending the fine needle-like or columnar crystals of taurine obtained in the step of growing crystals into the skin external agent composition.

## Description

### TECHNICAL FIELD

The present invention relates to a method for producing fine needle-like or columnar crystals of taurine and uses thereof. Specifically, the present invention relates to a skin external agent containing taurine, and relates to an increase in skin permeability and a pharmacological effect of taurine.

### BACKGROUND ART

Taurine is a compound having a chemical name of 2-aminoethanesulfonic acid, and is a sulfur-containing free amino acid having the highest content in living cells. Taurine is known to exhibit an action like vitamins and hormones or various pharmacological actions including a cranial nervous system, a circulatory system, and a hepatobiliary system, unlike amino acids constituting proteins, but it has recently been found that taurine plays an important role in moisture retention of skin.

As a method for producing taurine, many production methods such as extraction from marine products and a plurality of organic synthesis methods are known. For example, Patent Document 1 discloses a production method for obtaining taurine with high purity, and Patent Document 2 discloses a production method for obtaining taurine with high yield.

As a technique for purifying taurine, Patent Document 3 discloses a method for purifying taurine in which a solution containing taurine and an inorganic salt is brought into contact with a porous solid substance exhibiting a molecular sieving effect, the taurine is adsorbed to the porous solid substance to be separated from the inorganic salt, and then the taurine adsorbed to the porous solid substance is desorbed using an organic solvent which may contain water. In addition, as a means for purifying taurine in a high yield, a technique for performing crystallization by adding alcohol is conventionally known. That is, when taurine is purified from a hot water or warm water extraction solution of a natural product (marine product or the like) or a synthesis reaction end solution, taurine crystals are precipitated in an alcohol aqueous solution, and the taurine crystals are finally obtained by filtration and drying. Patent Document 4 discloses a method for purifying taurine in which impurities are separated or removed by adding and treating alcohol to a taurine-containing reaction liquid obtained through a production process. As a method for purifying the obtained crystal, Patent Document 5 discloses a method for purifying taurine in which a crystal precipitated by crystallization is separated from a crystallization mother liquor, and then the crystal is treated with an alcohol aqueous solution containing hydrochloric acid, followed by a treatment with an alcohol aqueous solution not containing hydrochloric acid.

### PRIOR ART DOCUMENT

### PATENT DOCUMENT

Patent Document 1: Japanese Patent Laid-open Publication No. 2021-172652
Patent Document 2: Japanese Patent Laid-open Publication No. 2021-063060
Patent Document 3: Japanese Patent Laid-open Publication No. 2005-232026
Patent Document 4: Japanese Patent Laid-open Publication No. H06-192209
Patent Document 5: Japanese Patent Laid-open Publication No. H07-017943

### SUMMARY OF THE INVENTION

### PROBLEM TO BE SOLVED BY THE INVENTION

In the conventional production method and purification method, a technique for obtaining a taurine crystal with high purity and high yield is shown, but a technique for controlling the size and shape of the crystal is not shown.

When taurine is contained in the form of crystals in the skin external agent, it can be expected that the fine needle-like crystals pierce the stratum corneum, which is a skin barrier, to form micropores, thereby improving the transdermal absorption of taurine itself and the drug in the external agent. When taurine is contained in the form of a crystal in a skin external agent, the use feeling greatly changes depending on the crystal size, and a crystal having a certain size or more gives a rough feeling at the time of use, resulting in an uncomfortable use feeling. Therefore, it has been desired to obtain a fine needle-like crystal of a specific size or less so as to obtain a smooth feel without skin feel at the time of use.

### MEANS FOR SOLVING THE PROBLEMS

In order to solve the problems described above, the present inventors have extensively conducted studies, and as a result, it has been found that it is possible to produce fine needle-like or columnar crystals of taurine with a controlled crystalline form by creating an extremely low solubility state by either adding an aqueous solution in which taurine is dissolved to a liquid substance having a low solubility of taurine or gently pouring a liquid substance having a low solubility of taurine into an aqueous solution in which taurine is dissolved to form an interface, and then proceeding recrystallization. It has been found that the fine needle-like or columnar crystals of taurine obtained by this method have a thickness in the range of 5 to 150 micrometers and a length in the range of 100 to 3000 micrometers, and in a skin external agent composition containing fine needle-like or columnar crystals of taurine, there is no rough feeling of crystals, and it is possible to provide an agent having smooth application feeling of fine needle-like or columnar crystals.

The present invention is as follows.
[1] A skin external agent composition containing fine needle-like or columnar crystals of taurine having a thickness of less than 150 micrometers and a length of less than 3000 micrometers.
[2] The skin external agent composition according to [1], further containing a useful substance.
[3] The skin external agent composition according to [1] or [2], wherein the fine needle-like or columnar crystals of taurine are grown in an environment in which an aqueous solution in which taurine is dissolved is mixed with or brought into contact with a liquid substance having low solubility of taurine.
[4] The skin external agent composition according to [3], wherein the liquid substance having low solubility of taurine is a water-soluble organic solvent.
[5] A method for producing a fine needle-like or columnar crystals of taurine, the method including a step of growing crystals in an environment in which an aqueous solution in which taurine is dissolved is mixed with or brought into contact with a liquid substance having low solubility of taurine.
[6] The method according to [5], wherein the taurine concentration in the aqueous solution is 3 to 28 mass%.
[7] The method according to [5] or [6], wherein an amount of the liquid substance having low solubility of taurine is 50 mass% or less with respect to a total amount of the aqueous solution in which taurine is dissolved.
[8] The method according to any one of [5] to [7], wherein the liquid substance having low solubility of taurine is a water-soluble organic solvent having a taurine solubility of 1.0 g or less per 100 g of the liquid substance (25°C).
[9] The method according to [8], wherein the water-soluble organic solvent is one kind or two or more kinds selected from the group consisting of ethanol, isopropyl alcohol, ethylene glycol, 1,2-propanediol, 1,3-propanediol, 1,2-butanediol, 1,3-butanediol, isoprene glycol, diethylene glycol, dipropylene glycol, diethylene glycol monoethyl ether, glycerin, diglycerin, triglycerin, 1,2-pentanediol, 1,2-hexanediol, cyclohexylglycerin, and n-hexylglycerin.
[10] A method for producing a skin external agent composition containing fine needle-like or columnar crystals of taurine, the method including the steps of: growing crystals in an environment in which an aqueous solution in which taurine is dissolved is mixed with or brought into contact with a liquid substance having low solubility of taurine; and blending the fine needle-like or columnar crystals of taurine obtained in the step of growing crystals into the skin external agent composition.
[11] The method according to [10], wherein the taurine concentration in the aqueous solution is 3 to 28 mass%.
[12] The method according to [10] or [11], wherein an amount of the liquid substance having low solubility of taurine is 50 mass% or less with respect to a total amount of the aqueous solution in which taurine is dissolved.
[13] The method according to [10], wherein the liquid substance having low solubility of taurine is a water-soluble organic solvent having a taurine solubility of 1.0 g or less per 100 g of the liquid substance (25°C).
[14] The method according to [13], wherein the water-soluble organic solvent is one kind or two or more kinds selected from the group consisting of ethanol, isopropyl alcohol, ethylene glycol, 1,2-propanediol, 1,3-propanediol, 1,2-butanediol, 1,3-butanediol, isoprene glycol, diethylene glycol, dipropylene glycol, diethylene glycol monoethyl ether, glycerin, diglycerin, triglycerin, 1,2-pentanediol, 1,2-hexanediol, cyclohexylglycerin, and n-hexylglycerin.
[15] The method according to [10], wherein the skin external agent composition is a liquid agent, lotion, emulsion, cream, or aqueous gel, and a taurine concentration in an aqueous phase in the blending step is maintained at a saturated concentration or higher, whereby the fine needle-like or columnar crystals of taurine are retained in the skin external agent composition.
[16] The method according to [10], wherein the skin external agent composition is a cosmetic oil or an ointment, and a product obtained by filtering and drying the fine needle-like or columnar crystals of taurine obtained in the step of growing crystals is blended in the skin external agent composition in the blending step.

### EFFECT OF THE INVENTION

Since the skin external agent composition of the present invention contains taurine whose crystal size and shape are controlled, the composition has a good touch and a smooth touch. Taurine has high polarity and low skin permeability, but when the taurine is applied to the skin by fine needle-like or columnar crystallization, the needle-like material enters the stratum corneum, thereby increasing the skin permeability of the taurine and increasing the pharmacological effect.

### BRIEF DESCRIPTION OF THE DRAWINGS

FIG. 1 is a photomicrograph showing an example of fine needle-like crystals of taurine.
FIG. 2 is a photomicrograph showing an example of massive crystals of a taurine raw material.

### MODE FOR CARRYING OUT THE INVENTION

The skin external agent composition of the present invention contains fine needle-like or columnar crystals of taurine having a thickness of less than 150 micrometers and a length of less than 3000 micrometers.

The fine needle-like or columnar crystals of taurine in the present invention can be produced by mixing an aqueous solution (A) in which the taurine is dissolved with a liquid substance (B) having low solubility of taurine (for example, adding the liquid substance (B) while stirring the aqueous solution (A)), or bringing the aqueous solution (A) in which the taurine is dissolved into contact with the liquid substance (B) having low solubility of taurine (for example, gently pouring the liquid substance (B) into the aqueous solution (A) to form an interface), for example, to create a state having extremely low solubility, and then allowing recrystallization to proceed (crystal growth). A crystalline form of the fine needle-like or columnar crystals of taurine to be obtained is controlled.

The taurine as a raw material used in the present invention is not particularly limited, and taurine conventionally used in pharmaceuticals, quasi-drugs, and cosmetics can be used, and any of synthetic products and extracts from natural products can be used.

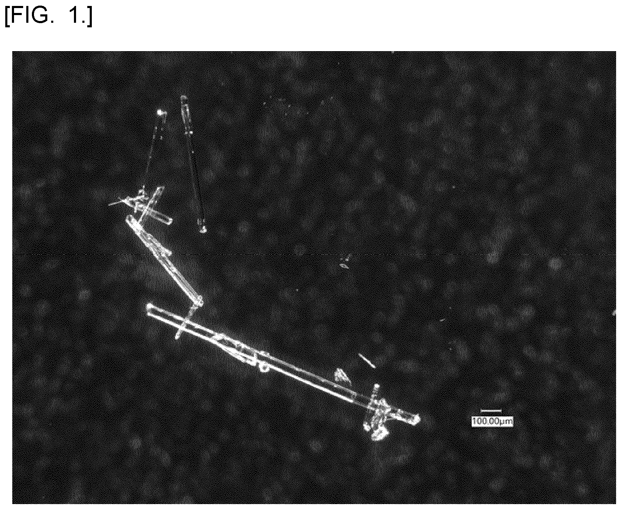

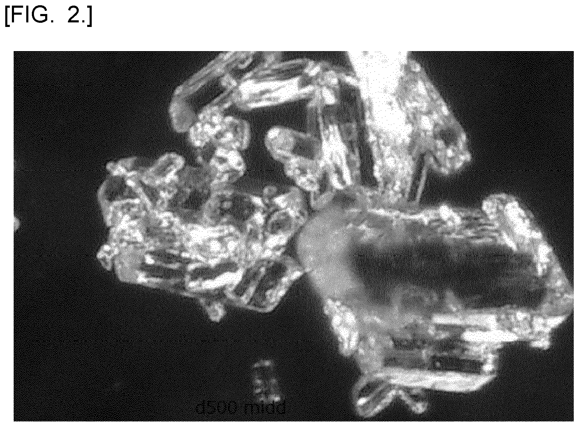

The fine needle-like or columnar crystals of taurine in the present invention may be needle-like crystals, columnar crystals, or a mixture thereof. Here, the needle-like crystal refers to a shape in which a major axis of the crystal is three times or more a minor axis, and the columnar crystal refers to a shape in which the major axis of the crystal is less than three times the minor axis. The fine needle-like or columnar crystal of taurine in the present invention is a crystalline form that can be distinguished from the massive crystal of the raw material taurine by microscopic observation. An example of the fine needle-like crystals is illustrated in FIG. 1, and an example of the massive crystals is illustrated in FIG. 2.

The size of the fine needle-like crystal or columnar crystal of taurine is preferably in the range of a thickness of 5 to 150 micrometers and a length of 100 to 3000 micrometers, more preferably in the range of a thickness of 10 to 100 micrometers and a length of 100 to 2000 micrometers, and most preferably in the range of a thickness of 10 to 60 micrometers and a length of 150 to 2000 micrometers, from the viewpoint of imparting a smooth application feeling without leaving a rough feeling when used by being contained in the skin external agent composition.

Here, the thickness is the minor axis of the crystal, and the length is the major axis of the crystal.

A crystalline form having a thickness of 150 micrometers and a length of more than 3000 micrometers (for example, taurine crystal and taurine crystalline powder which are commercially available as raw materials) is not preferable because a rough foreign body feeling remains on the skin when applied.

In the present invention, fine crystals having a thickness of less than 5 micrometers and a length of less than 100 micrometers may be contained in the fine crystals of taurine, but it is particularly desirable that the fine crystals do not contain crystals having a thickness of more than 150 micrometers. According to the method for producing fine needle-like crystals or columnar crystals of taurine of the present invention, recrystallization conditions can be set so that needle-like crystals or columnar crystals having a thickness exceeding 150 micrometers are not generated.

The specific recrystallization (crystal growth) condition is a step of growing a crystal under an environment in which an aqueous solution (A) in which the taurine is dissolved is mixed with or brought into contact with a liquid substance (B) having low solubility of taurine.

Examples of the liquid substance (B) having low solubility of taurine, which is used for recrystallization (crystal growth) include organic solvents, but are not particularly limited to, and polar organic solvents such as acetone, methyl ethyl ketone, dimethyl sulfoxide, dimethylformamide, ethyl acetate, isopropyl myristate, methyl heptyl laurate, and jojoba oil; nonpolar organic solvents such as dodecane, isododecane, liquid paraffin, squalane, hydrogenated polyisobutene, and olefin oligomers; alcohols such as ethanol and 1-propanol; and polyhydric alcohols such as 1,3-butanediol, glycerin, and 1,2-pentanediol. Among them, since the effect of lowering the solubility of taurine is high by addition, the liquid substance (B) is preferably a liquid substance (water-soluble organic solvent) having a solubility in water at 25°C of 1 mass% or more, and more preferably a water-soluble organic solvent having a taurine solubility of 1.0 g or less per 100 g (25°C). Specifically, the liquid substance (B) is preferably one kind or two or more kinds selected from the group consisting of ethanol, 1-propanol, 2-propanol, 1-butanol, 2-butanol, ethylene glycol, diethylene glycol, 1,2-propanediol, 1,3-propanediol, 1,2-butanediol, 1,3-butanediol, isoprene glycol, diethylene glycol, dipropylene glycol, diethylene glycol monoethyl ether, glycerin, diglycerin, triglycerin, 1,2-pentanediol, 1,2-hexanediol, acetone, methyl ethyl ketone, dimethyl sulfoxide, dimethylformamide, cyclohexylglycerin, and n-hexylglycerin, and from the viewpoint of using the obtained fine needle crystals as a component of a skin external agent, it is most preferably one kind or two or more kinds selected from the group consisting ethanol, isopropyl alcohol, ethylene glycol, 1,2-propanediol, 1,3-propanediol, 1,2-butanediol, 1,3-butanediol, isoprene glycol, diethylene glycol, dipropylene glycol, diethylene glycol monoethyl ether, glycerin, diglycerin, triglycerin, 1,2-pentanediol, 1,2-hexanediol, cyclohexylglycerin, and n-hexylglycerin. In the case of two or more kinds, those can be used as a mixed solvent.

When the liquid substance (B) having a low solubility of taurine is not used, the crystalline form does not become a fine needle-like or columnar crystal, and when crystals are blended in a skin external agent composition, the skin external agent composition has rough use feeling, which is not preferable.

The taurine concentration in the aqueous solution (A) in which taurine used for recrystallization is dissolved is preferably 3 mass% or more from the viewpoint of forming fine needle-like crystals when mixed with a solvent having low solubility of taurine, and is preferably 28 mass% or less from the viewpoint of the solubility of taurine in water. When the content is less than 3 mass%, crystals are not precipitated unless the liquid substance (B) is added in an amount of 100 mass% or more with respect to the aqueous solution (A), and a sufficient amount of crystals cannot be obtained, which is not realistic in terms of cost and yield. When the content is more than 28 mass%, it is difficult to dissolve taurine even when the mixture is heated to 80°C or higher, so that the step of recrystallization is not realistic. Thus, the taurine concentration in the aqueous solution (A) is usually 5 to 20 mass%, preferably 6 to 18 mass%, and optimally 7 to 15 mass%.

It is possible to add one or more water-soluble organic solvents having low solubility of taurine to the aqueous solution (A) in which taurine used for recrystallization is dissolved for the purpose of lowering the saturated concentration. As the water-soluble organic solvent in this case, glycerin, 1,3-propanediol, ethanol, isopropyl alcohol, ethylene glycol, 1,2-propanediol, 1,2-butanediol, 1,3-butanediol, isoprene glycol, diethylene glycol, dipropylene glycol, diethylene glycol monoethyl ether, diglycerin, triglycerin, 1,2-pentanediol, 1,2-hexanediol, cyclohexylglycerin, n-hexylglycerin, and the like are preferable. The concentration of the water-soluble organic solvent is usually 50 mass% or less, preferably 30 mass% or less, and optimally 25 mass% or less in the aqueous solution (A) in which taurine is dissolved. If it exceeds 50 mass%, the solubility of taurine becomes too low and a sufficient amount of crystals cannot be obtained, which is not realistic in terms of cost and yield.

The fine needle-like or columnar crystals of taurine obtained in the present invention may be obtained by filtration and drying, and may be blended in a skin external agent, or a solution in which fine needle-like or columnar crystals are precipitated may be blended as it is.

The skin external agent composition of the present invention can be used mainly as an external composition for cosmetics and quasi-drugs. The dosage form of the composition of the present invention is not particularly limited, and examples thereof include liquid agents, lotions, cosmetic oils, emulsions, creams, aqueous gels, and ointments. These agents can be prepared by a conventional method.

For the liquid agents, lotions, emulsions, creams, and aqueous gel agents, it is possible to prepare an agent while maintaining the fine needle-like or columnar crystals by maintaining the taurine concentration in the aqueous phase at a saturated concentration or more.

With regard to the cosmetic oils and the ointments, the fine needle-like crystal can be prepared by blending those obtained by filtration and drying.

In addition, the skin external agent composition of the present invention may contain a component that is usually used in agents such as cosmetics, quasi-drugs, and external pharmaceuticals as long as the effect of the present invention is not impaired. In the present specification, these components are referred to as useful substances. The skin external agent composition of the present invention preferably contains a useful substance. Examples of the useful substances include water, oil agents, surfactants, gelling agents, powders, alcohols, water-soluble polymers, film-forming agents, resins, ultraviolet protecting agents, inclusion compounds, antibacterial agents, fragrances, deodorants, salts, pH adjusting agents, refreshing agents, extracts derived from animals and microorganisms, plant extracts, blood circulation promoters, astringents, antiseborrheic agents, whitening agents, anti-inflammatory agents, moisturizers, keratolytic agents, enzymes, hormones, and vitamins. One or more kinds of the useful substances can be used.

The water contained in the skin external agent composition of the present invention is not particularly limited, and examples thereof include purified water, ion-exchanged water, and tap water.

Examples of the water-soluble alcohol include lower alcohols, polyhydric alcohols, polyhydric alcohol polymers, dihydric alcohol alkyl ethers, dihydric alcohol alkyl ethers, dihydric alcohol ether esters, glycerin monoalkyl ethers, sugar alcohols, monosaccharides, oligosaccharides, polysaccharides, and derivatives thereof.

Examples of the lower alcohol include ethanol, propanol, isopropanol, isobutyl alcohol, and t-butyl alcohol.

Examples of the polyhydric alcohol include dihydric alcohols (for example, dipropylene glycol, 1,3-butylene glycol, ethylene glycol, trimethylene glycol, 1,2-butylene glycol, tetramethylene glycol, 2,3-butylene glycol, pentamethylene glycol, 2-butene-1,4-diol, hexylene glycol, octylene glycol, and the like), trihydric alcohols (for example, glycerin, trimethylolpropane, and the like), tetrahydric alcohols (for example, diglycerin, pentaerythritol such as 1,2,6-hexanetriol, and the like), pentahydric alcohols (for example, xylitol, triglycerin, and the like), hexahydric alcohols (for example, sorbitol, mannitol, and the like), polyhydric alcohol polymers (for example, diethylene glycol, dipropylene glycol triethylene glycol, polypropylene glycol, tetraethylene glycol, diglycerin-triglycerin, tetraglycerin, polyglycerin, and the like), dihydric alcohol alkyl ethers (for example, include ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether, and the like), dihydric alcohol alkyl ethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether triethylene glycol monomethyl ether triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, and the like), dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadibate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, and the like), glycerin monoalkyl ethers (for example, xyl alcohol, ceracyl alcohol, batil alcohol, and the like), sugar alcohols (for example, maltotriose, mannitol, sucrose, erythritol, glucose, fructose, starch degrading sugar, maltose, starch degrading sugar reducing alcohol, and the like), glysolid, tetrahydrofurfuryl alcohol, POE-tetrahydrofuryl alcohol, POP-butyl ether, POP-POE-butyl-tertripolyoxypropylene glycerin ether, POP-glycerin ether, POP-glycerin ether phosphoric acid, POP-POE-pentaerythritol ether, and polyglycerin.

Examples of the monosaccharide include tricarbonate (for example, D-glycerylaldehyde, dihydroxyacetone, and the like), tetracarbonate (for example, D-erythrose, D-erythrulose, D-treose, erythritol, and the like), pentacarbonate (for example, L-arabinose, D-xylose, L-lyxose, D-arabinose, D-ribose, D-Ribulose, D-xylulose, L-xylulose, and the like), hexacarbonate (for example, D-glucose, D-talose, D-busicose, D-galactose, D-fructose, L-galactose, L-mannose, D-tagatose, and the like), heptacarbonate (for example, aldoheptos, heppros, and the like), octacarbonate (for example, octulose or the like), deoxysugar (for example, 2-deoxy-D-ribose, 6-deoxy-L-galactose, 6-deoxy-L-mannose, and the like), amino (for example, D-glucosamine, D-galactosamine, sialic acid, aminouronic acid, muramic acid, and the like), and uronic (for example, D-glucuronic acid, D-mannuronic acid, L-guluronic acid, D-galacturonic acid, L-iduronic acid, and the like).

Examples of the oligosaccharide include sucrose, gentianose, umbelliferose, lactose, planteose, isoliucnoses, α,α-trehalose, raffinose, rixnoses, unbilicin, and stachyose velvascose.

Examples of the polysaccharide include cellulose, quince seed, starch, galactan, dermatan sulfate, glycogen, gum arabic, heparan sulfate-tragacanth gum, keratan sulfate, chondroitin, xanthan gum, guar gum, dextran, keratosulfate, locust bean gum, and succinoglucan.

Examples of the anti-inflammatory agent include plant-derived components, allantoin and derivatives thereof, glycyrrhetinic acid and derivatives thereof, glycyrrhizic acid and salts or derivatives thereof, salicylic acid derivatives, aminocaproic acid, azulene, and derivatives thereof.

Examples of the gelling agent (thickener) include gum arabic, carrageenan, gum karaya, gum tragacanth, carob gum, quince seed (marmelo), casein, dextrin, gelatin, sodium pectinate, sodium araginate, methyl cellulose, ethyl cellulose, CMC, hydroxyethyl cellulose, hydroxypropyl cellulose, PVA, PVM, PVP, sodium polyacrylate, a carboxyvinyl polymer, locust bean gum, guar gum, tamarind gum, dialkyldimethylammonium sulfate cellulose, xanthan gum, aluminum magnesium silicate, bentonite, hectorite, A1Mg silicate (VEEGUM), laponite, and silicic anhydride.

Examples of the natural water-soluble polymer include plant-based polymers (for example, gum arabic, gum tragacanth, galactan, guar gum, carob gum, gum karaya, carrageenan, pectin, agar, quince seed (marmelo), algae colloid (brown algae extract), starch (rice, corn, potato, wheat), glycyrrhizic acid, and the like), microorganism-based polymers (for example, xanthan gum, dextran, succinoglucan, pullulan, and the like), and animal-based polymers (for example, collagen, casein, albumin, gelatin, and the like).

Examples of the semi-synthetic water-soluble polymer include starch-based polymers (for example, carboxymethyl starch, methyl hydroxypropyl starch, and the like), cellulose-based polymers (methyl cellulose, ethyl cellulose, methyl hydroxypropyl cellulose, hydroxyethyl cellulose, sodium cellulose sulfate, hydroxypropyl cellulose, carboxymethyl cellulose, sodium carboxymethyl cellulose, crystalline cellulose, cellulose powder, and the like), and alginic acid-based polymers (for example, sodium alginate, propylene glycol alginate ester, and the like).

Examples of the synthetic water-soluble polymer include vinyl-based polymers (for example, polyvinyl alcohol, polyvinyl methyl ether, polyvinyl pyrrolidone, carboxyvinyl polymer, and the like), polyoxyethylene-based polymers (for example, polyethylene glycol 20,000, 40,000, 60,000, and the like), acryl-based polymers (for example, sodium polyacrylate, polyethyl acrylate, polyacrylamide, and the like), polyethyleneimine, and a cationic polymer.

Examples of the moisturizer include chondroitin sulfate, hyaluronic acid, mucoitin sulfate, charonic acid, atelocollagen, cholesteryl-12-hydroxystearate, sodium lactate, bile acid salt, DL-pyrrolidone carboxylate, short-chain soluble collagen, a diglycerin (EO) PO adduct, a chestnut rose extract, a yarrow extract, and a melilot extract.

Examples of the whitening agent (whitening component) include tranexamic acid, ascorbic acid and salts thereof, vitamin C such as ascorbic acid derivatives (sodium ascorbate phosphate, magnesium ascorbate phosphate, ascorbyl tetra2-hexyldecanoate, 2-O-ethyl ascorbic acid, 3-O-ethyl ascorbic acid, ascorbic acid glucoside, and the like), arbutin, kojic acid, placenta, ellagic acid, nicotinic acid amide, hydroquinone, and linoleic acid and derivatives thereof.

Examples of the keratolytic agent (keratin flexible component) include lactic acid, salicylic acid, gluconic acid, glycolic acid, citric acid, malic acid, fruit acid, phytic acid, urea, and sulfur.

Examples of the anti-aging component include hydrolyzed soybean protein, retinoid (Retinol and derivatives thereof, retinoic acid, retinal, and the like), kinetin, adenosine, NMN (nicotinamide mononucleotide), AMP (adenosine monophosphate), ADP (adenosine diphosphate), ATP (adenosine triphosphate), ursolic acid, a turmeric extract, and a sphingosine derivative, mevalonolactone.

Examples of the anti-glycation component include plant extracts such as a Buddleja axillaris leaf extract, evening primrose oil, fruit or fruit juice of amla or extract thereof, L-arginine, L-lysine, hydrolyzed casein, hydrolyzable tannin, and carnosine.

Examples of the blood circulation promoter (blood circulation promoting action component) include the component derived from a plant such as Asian ginseng, Angelica keiskei, mountain arnica, ginkgo, fennel, Isodonis japonicus, Dutch oak, chamomile, Roman chamomile, Daucus carota sativa, gentian, burdock, rice, Japanese hawthorn, shiitake mushroom, ginger, English hawthorn, Juniperus communis, Cnidium officinale, swertia herb, thyme, clove, citrus unshiu peel, capsicum, Angelica acutiloba, peach kernel, spruce, carrot, garlic, butcher's bloom, grape, peony, horse chestnut, lemon balm, yuzu, coix seed, green tea, rosemary, rosehip, citrus unshiu peel, Angelica acutiloba, peach kernel, peach, apricot, walnut, corn, golden meal, ichthammol, cantharides tincture, cepharanthine: gamma oryzanol, tocopherol nicotinate, and glucosyl hesperidin.

Examples of the polyphenols include flavonoid-based polyphenols such as curcuminoids, flavanones, stilpenoids, polymethoxyflavonoids, flavonols, xanthonoids, chalcones, lignoids, flavanols, and isoflavones.

Examples of the sequestering agent include 1-hydroxyethane -1,1-diphosphonic acid, 1-hydroxyethane-1,1-diphosphonic acid tetrasodium salt, edetate disodium, edetate trisodium, edetate tetrasodium, citrate sodium, polyphosphate sodium, metaphosphate sodium, gluconic acid, phosphoric acid, citric acid, ascorbic acid, succinic acid, edetic acid, and ethylenediamine hydroxyethyl triacetate trisodium.

Examples of the ultraviolet protecting agent (water-soluble ultraviolet absorber) include benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone, benzimidazole-based ultraviolet absorbers such as phenylbenzimidazole-5-sulfonic acid and salts thereof, and phenylene-bis-benzimidazole-tetrasulfonic acid and salts thereof, 3-(4'-methylbenzylidene)-d, l-camphor, 3-benzylidene-d, l-camphor, urocanic acid, and urocanic acid ethyl ester.

Examples of the powder (powder component) include inorganic powders (for example, talc, kaolin, mica, sericite, muscovite, phlogopite, synthetic mica, lepidolite, biotite, permiculite, magnesium carbonate, calcium carbonate, aluminum silicate, barium silicate, calcium silicate, magnesium silicate, strontium silicate, metal tungstate, magnesium, silica, zeolite, barium sulfate, calcined calcium sulfate (calcined gypsum), calcium phosphate, fluorapatite, hydroxyapatite, ceramic powder, metal soap (for example, zinc myristate, calcium palmitate, and aluminum stearate), boron nitride, and the like), organic powders (for example, polyamide resin powder (nylon powder), polyethylene powder, polymethyl methacrylate powder, polystyrene powder, copolymer resin powder of styrene and acrylic acid, benzoguanamine resin powder, polytetrafluoroethylene powder, cellulose powder, and the like), inorganic white pigments (for example, titanium dioxide, zinc oxide, and the like), inorganic red pigments (for example, iron oxide (red iron oxide), iron titanate, and the like), inorganic brown pigments (for example, γ-iron oxide or the like), inorganic yellow pigments (for example, yellow iron oxide, yellow earth, and the like), inorganic black pigments (for example, black iron oxide, low order titanium oxide, and the like), inorganic violet pigments (for example, mango violet, cobalt violet, and the like), inorganic green pigments (for example, chromium oxide, chromium hydroxide, cobalt titanate, and the like), inorganic blue pigments (for example, ultramarine blue, Prussian blue, and the like), pearl pigments (for example, titanium oxide coated mica, titanium oxide coated bismuth oxychloride, titanium oxide coated talc, colored titanium oxide coated mica, bismuth oxychloride, fish scale foil, and the like), metal powder pigments (for example, aluminum powder, copper powder, or the like), organic pigments such as zirconium, barium or aluminum lake (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1, and the like), and natural pigments (for example, chlorophyll, β-carotene, and the like).

Examples of the vitamins include vitamins A, B1, B2, B6, C, and E and derivatives thereof, pantothenic acid and derivatives thereof, and biotin.

Examples of amino acids include neutral amino acids such as glycine, alanine, valine, leucine, and isoleucine, aliphatic amino acids and oxyamino acids such as serine and threonine, aliphatic amino acids and sulfur-containing amino acids such as cysteine, cystine, and methionine, acidic amino acids such as glutamine and asparagine, basic amino acid such as arginine, aromatic amino acids such as phenylalanine and tyrosine, and cyclic amino acids such as proline and oxyproline.

Examples of the peptide include human oligopeptide, tripeptide, acetyl hexapeptide, palmitopentapeptide, and collagen peptide.

Examples of the antioxidant include tocopherols, dibutylhydroxytoluene, butylhydroxyanisole, and gallic acid esters.

Examples of the pH adjusting agent include buffers such as sodium lactate, sodium citrate, and sodium succinate.

Examples of the oil agent (oil phase component) include liquid oils-and-fats, solid oils-and-fats, waxes, hydrocarbon oils, higher fatty acids, synthetic ester oils, silicone oils and the like which are usually used in cosmetics and quasi-drugs. These oil phase components and the aqueous phase components described above can be combined with an appropriate surfactant to form a skin composition.

Examples of the liquid oil-and-fat include avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, cinnamon oil, Japanese tung oil, jojoba oil, germ oil, and triglycerin.

Examples of the solid oil-and-fat include cocoa butter, coconut oil, horse fat, hardened coconut oil, palm oil, beef tallow, mutton fat, hardened beef tallow, palm kernel oil, lard, beef bone fat, Japan wax kernel oil, hardened oil, beef leg fat, Japan wax, and hardened castor oil.

Examples of waxes include beeswax, candelilla wax, cotton wax, carnauba wax, bayberry wax, chinese wax, spermaceti wax, montan wax, rice bran wax, lanolin, kapok wax, lanolin acetate, liquid lanolin, sugarcane wax, lanolin fatty acid isopropyl, hexyl laurate, reduced lanolin, jojoba wax, hard lanolin, shellac wax, polyoxyethylene lanolin alcohol ether, polyoxyethylene lanolin alcohol acetate, polyoxyethylene cholesterol ether, lanolin fatty acid polyethylene glycol, polyoxyethylene hydrogenated lanolin alcohol ether, and cetyl palmitate.

Examples of the hydrocarbon oil include liquid paraffin, ozokerite, squalane, pristane, paraffin, ceresin, squalene, petrolatum, and microcrystalline wax.

Examples of the higher fatty acid include lauric acid, myristic acid, palmitic acid, stearic acid, behenic acid, oleic acid, undecylenic acid, tall oil acid, linoleic acid, linoleic acid, eicosapentaenoic acid (EPA), and docosahexaenoic acid (DHA).

Examples of the synthetic ester oil include cetyl octanoate, myristyl myristate, glyceryl tri2-ethylhexanoate, pentaerythritol tetra2-ethylhexanoate, dioctyl succinate, and tripropylene glycol dineopentanoate.

Examples of the silicone oil include chain polysiloxane (for example, dimethylpolysiloxane, methylphenylpolysiloxane, diphenylpolysiloxane, and the like); cyclic polysiloxanes (for example, octamethylcyclotetrasiloxane, decamethylcyclopentasiloxane, dodecamethylcyclohexasiloxane, and the like), silicone resins forming a three-dimensional network structure, silicone rubbers, various modified polysiloxanes (amino-modified polysiloxane, polyether-modified polysiloxane, alkyl-modified polysiloxane, fluorine-modified polysiloxane, and the like), and acrylic silicones.

### EXAMPLES

Hereinafter, the present invention will be described in detail with reference to Examples, Comparative Examples, and Test Examples, but the present invention is not limited to the following examples.

The blending amount in the following is mass% unless otherwise specified, and the unit of thickness and length is micrometers.

### Comparative Example 1

Commercially available natural taurine (Product name: Taurine (extract), manufactured by Chori Co., Ltd.) was used.

### Comparative Example 2

Commercially available synthetic taurine (Product name: Taurine of the Japanese Pharmacopoeia, manufactured by Shoka Pharmacy Co., Ltd.) was used.

### Comparative Example 3

An aqueous solution containing 10% of taurine was heated to 50°C and uniformly dissolved, then cooled to room temperature, and allowed to stand for 24 hours. The precipitated crystals were filtered off and dried to obtain crystals.

### Example 1

An aqueous solution containing 10% of taurine and 10% of 1,3-propanediol was heated to 50°C and uniformly dissolved, and then cooled to 30°C while being stirred with a disperser mixer. The precipitated crystals were filtered off, washed with ethanol, and then dried to obtain crystals.

### Example 2

An aqueous solution containing 10% of taurine was heated to 50°C and uniformly dissolved, then cooled to 30°C with stirring, and 10% of ethanol was added to the total amount of the solution in which taurine was dissolved while stirring. The precipitated crystals were filtered off, washed with ethanol, and then dried to obtain crystals.

### Example 3

An aqueous solution containing 10% of taurine was heated to 50°C and uniformly dissolved, and then cooled to 30°C with stirring, and 10% of 1,3-butanediol was added to the total amount of the solution in which taurine was dissolved while stirring. The precipitated crystals were filtered off, washed with ethanol, and then dried to obtain crystals.

### Example 4

An aqueous solution containing 10% of taurine and 10% glycerin was heated to 50°C and uniformly dissolved, then cooled to 30°C with stirring, and 10% of ethanol was added to the total amount of the solution in which taurine was dissolved while stirring. The precipitated crystals were filtered off, washed with ethanol, and then dried to obtain crystals.

### Example 5

An aqueous solution containing 10% of taurine was heated to 50°C and uniformly dissolved, then cooled to 30°C with stirring, and allowed to stand, and then 10% of ethanol was slowly poured with respect to the total amount of the solution in which taurine was dissolved while stirring. The precipitated crystals at the interface separated into two phases were filtered off, washed with ethanol, and then dried to obtain crystals.

### Example 6

An aqueous solution containing 10% of taurine was heated to 50°C and uniformly dissolved, and then a solution cooled to 30°C with stirring was poured into 10% of ethanol with respect to the total amount of the solution in which taurine was dissolved while stirring. The precipitated crystals were filtered off, washed with ethanol, and then dried to obtain crystals.

### Example 7

An aqueous solution containing 10% of taurine and 10% of glycerin was heated to 50°C and uniformly dissolved, and then a solution cooled to 30°C with stirring was poured into 10% of ethanol with respect to the total amount of the solution in which taurine was dissolved while stirring. The precipitated crystals were filtered off, washed with ethanol, and then dried to obtain crystals.

### Example 8

An aqueous solution containing 10% of taurine and 10% of glycerin was heated to 50°C and uniformly dissolved, and then a solution cooled to 30°C with stirring was poured into 10% of 1,3-propanediol with respect to the total amount of the solution in which taurine was dissolved while stirring. The precipitated crystals were filtered off, washed with ethanol, and then dried to obtain crystals.

### Example 9

An aqueous solution containing 10% of taurine and 10% of glycerin was heated to 50°C and uniformly dissolved, and then the solution cooled to 30°C with stirring was poured into 3% of 1,2-pentanediol with respect to the total amount of the solution in which taurine was dissolved while stirring. The precipitated crystals were filtered off, washed with ethanol, and then dried to obtain crystals.

### Example 10

An aqueous solution containing 18% of taurine was heated to 65°C and uniformly dissolved, and then poured into 10% of 1,3 propanediol with respect to the total amount of the solution in which taurine was dissolved while being stirred. The precipitated crystals were filtered off, washed with ethanol, and then dried to obtain crystals.

### Example 11

An aqueous solution containing 18% of taurine was heated to 65°C and uniformly dissolved, and then the solution was poured into a mixed solution of 5% of 1,3-propanediol and 5% of diethylene glycol monomethyl ether with respect to the total amount of the solution in which taurine was dissolved while being stirred. The precipitated crystals were filtered off, washed with ethanol, and then dried to obtain crystals.

### Test Example 1

The crystals of Comparative Examples 1 to 3 and the crystals obtained in Examples 1 to 11 were observed with a microscope. The crystalline form, thickness and length of the crystal are shown in Tables 4 to 6.

### Production Example 1

The crystals of Comparative Examples 1 to 3 and the crystals obtained in Examples 1 to 11 were blended into emulsion formulations shown in Table 1 to prepare emulsions.

The taurine concentration in a phase A was set to a saturated concentration at 25°C, a phase B was added to the phase A heated to 80°C while being mixed by a homomixer, the mixture was cooled to 25°C, a phase C was then mixed, and a taurine crystal of a layer D was mixed with the obtained composition to prepare an emulsion containing a crystal.

### Production Example 2

The crystals of Comparative Examples 1 to 3 and the crystals obtained in Examples 1 to 11 were blended in cosmetic oil formulations shown in Table 2 to prepare cosmetic oils.

The phase A was heated to 100°C, uniformly mixed, then cooled to 25°C, and the taurine crystal of layer B was mixed to prepare a cosmetic oil containing a crystal.

**[Table 1]**

| | Components | mass% |
|---|---|---|
| Phase A | Purified water | 61.81 |
| | 1,3-propanediol | 15.00 |
| | Taurine | 6.00 |
| | Glycerin | 5.00 |
| | Pentylene glycol | 1.50 |
| | (Hydroxyethyl acrylate/ Na acryloyldimethyltaurine) copolymer | 1.88 |
| | Isohexadecane | 1.13 |
| | Polysorbate 80 | 0.38 |
| Phase B | Squalane | 5.00 |
| Phase C | Phenoxyethanol | 0.30 |
| Phase D | Taurine crystals obtained in Comparative Examples and Examples | 2.00 |

**[Table 2]**

| | Components | mass% |
|---|---|---|
| Phase A | Squalane | 62.90 |
| | Hydrogenated polydecene | 29.00 |
| | Hydrogenated (styrene/isoprene) copolymer | 7.00 |
| | Tocopherol | 0.10 |
| Phase B | Taurine crystals obtained in Comparative Examples and Examples | 1.00 |

Sensory evaluation concerning the use feeling of the prepared emulsions and cosmetic oils was performed by five panelists. Each item was evaluated according to the criteria shown in Table 3, and the average score was used as a score. The results are shown in Tables 4 to 6.

**[Table 3]**

| | |
|---|---|
| 0 points | Always feel strong rough feeling |
| 2 points | Always feel weak rough feeling |
| 4 points | Sometimes feel weak rough feeling |
| 6 points | Very rarely feel weak rough feeling |
| 8 points | Feel smooth feeling |
| 10 points | Feel very smooth feeling |

**[Table 4]**

| | Comparative Example 1 | Comparative Example 2 | Comparative Example 3 | Example 1 | Example 2 | Example 3 |
|---|---|---|---|---|---|---|
| Crystalline form | Massive | Massive | Massive | Columnar | Columnar | Columnar |
| Maximum particle size (thickness) µm | 160 | 300 | 1680 | 145 | 80 | 100 |
| Maximum particle size (length) µm | 700 | 2000 | 6740 | 280 | 150 | 150 |
| Use feeling of emulsions (rating) | 3.2 | 2.8 | 0.0 | 8.4 | 9.2 | 8.8 |
| Use feeling of cosmetic oils (rating) | 2.8 | 1.2 | 0.0 | 8.0 | 9.6 | 8.8 |

**[Table 5]**

| | Example 4 | Example 5 | Example 6 | Example 7 |
|---|---|---|---|---|
| Crystalline form | Columnar | Needle-like | Needle-like | Needle-like |
| Maximum particle size (thickness) µm | 70 | 55 | 25 | 20 |
| Maximum particle size (length) µm | 100 | 800 | 250 | 250 |
| Use feeling of emulsions (rating) | 9.2 | 9.2 | 9.6 | 9.6 |
| Use feeling of cosmetic oils (rating) | 9.2 | 8.8 | 9.6 | 10.0 |

**[Table 6]**

| | Example 8 | Example 9 | Example 10 | Example 11 |
|---|---|---|---|---|
| Crystalline form | Needle-like | Needle-like | Needle-like | Needle-like |
| Maximum particle size (thickness) µm | 30 | 40 | 25 | 20 |
| Maximum particle size (length) µm | 220 | 150 | 720 | 370 |
| Use feeling of emulsions (rating) | 9.6 | 9.6 | 9.8 | 10.0 |
| Use feeling of cosmetic oils (rating) | 9.6 | 9.2 | 9.8 | 10.0 |

As shown in Tables 4 to 6, in the taurine crystal obtained by the production method of the present invention, it was confirmed that no roughness was observed in the use feeling of the compounded product due to the control of the crystal size, and the use feeling was clearly improved.

Formulation examples are shown below, but the present invention is not limited to these examples.

### Skin lotion

[A]

| | |
|---|---|
| Purified water | 71.70 mass% |
| Taurine | 8.00 mass% |
| Glycerin | 5.00 mass% |
| Diglycerin | 0.50 mass% |
| Betaine | 0.50 mass% |
| Phenoxyethanol | 0.30 mass% |

[B]

| | |
|---|---|
| Dipropylene glycol | 10.00 mass% |
| 1,2-pentanediol | 2.50 mass% |

[C]

| | |
|---|---|
| (Acrylates/steareth-20 methacrylate) copolymer | 0.45 mass% |
| Purified water | 1.05 mass% |

The mixture was heated to 80°C, the uniformly dissolved phase A was cooled to 35°C, added to the phase B with stirring, and stirred at 35°C for 10 minutes to precipitate fine needle-like crystals of taurine. Phase C was added to the mixture, and the mixture was stirred with a homomixer for 10 minutes and uniformly mixed to obtain an emulsion composition.

The shape of the fine crystals of taurine in the obtained composition was needle-like, with a maximum thickness of 50 micrometers and a maximum length of 750 micrometers. The use feeling of the obtained composition was smooth, and a composition having good use feeling was obtained.

### Aqueous gel

[A]

| | |
|---|---|
| Purified water | 71.7 mass% |
| Taurine | 8.00 mass% |
| Glycerin | 5.00 mass% |
| Raffinose | 0.50 mass% |
| Phenoxyethanol | 0.30 mass% |

[B]

| | |
|---|---|
| Dipropylene glycol | 10.00 mass% |
| 1,2-pentanediol | 2.50 mass% |

[C]

| | |
|---|---|
| Polyacrylate Crosspolymer-6 | 2.00 mass% |

The mixture was heated to 80°C, the uniformly dissolved phase A was cooled to 35°C, added to the phase B with stirring, and stirred at 35°C for 10 minutes to precipitate fine needle-like crystals of taurine. Phase C was added to the mixture, and the mixture was stirred with a homomixer for 10 minutes and uniformly mixed to obtain an emulsion composition.

The shape of the fine crystals of taurine in the obtained composition was needle-like, with a maximum thickness of 50 micrometers and a maximum length of 750 micrometers. The use feeling of the obtained composition was smooth, and a composition having good use feeling was obtained.

### Emulsion

[A]

| | |
|---|---|
| Purified water | 63.20 mass% |
| Taurine | 8.00 mass% |
| Glycerin | 5.00 mass% |

[B]

| | |
|---|---|
| Propanediol | 15.00 mass% |
| Pentylene glycol | 1.50 mass% |
| Phenoxyethanol | 0.30 mass% |

[C]

| | |
|---|---|
| (Hydroxyethyl acrylate/Na acryloyldimethyltaurine) | |
| copolymer | 0.75 mass% |
| Isohexadecane | 0.45 mass% |
| Polysorbate 80 | 0.15 mass% |
| Purified water | 0.65 mass% |

[D]

| | |
|---|---|
| Squalane | 5.00 mass% |

The mixture was heated to 80°C, the uniformly dissolved phase A was cooled to 35°C, added to the phase B with stirring, and stirred at 35°C for 10 minutes to precipitate fine needle-like crystals of taurine. A mixture of the phase C was added to the obtained mixture, and the mixture was stirred with a homomixer for 5 minutes and uniformly mixed. Then, the phase D was added, and the mixture was stirred with a homomixer for 10 minutes and emulsified to obtain an emulsion composition.

The shape of the fine crystals of taurine in the obtained composition was needle-like, with a maximum thickness of 30 micrometers and a maximum of 250 micrometers. The use feeling of the obtained composition was smooth, and a composition having good use feeling was obtained.

### Cream

[A]

| | |
|---|---|
| Purified water | 68.00 mass% |
| Taurine | 8.00 mass% |
| Glycerin | 5.00 mass% |

[B]

| | |
|---|---|
| 1,3-propanediol | 15.00 mass% |
| Pentylene glycol | 1.50 mass% |
| Phenoxyethanol | 0.30 mass% |

[C] (Hydroxyethyl acrylate/Na acryloyldimethyltaurine)

| | |
|---|---|
| copolymer | 0.75 mass% |
| Polysorbate 80 | 0.15 mass% |
| Purified water | 0.65 mass% |

[D]

| | |
|---|---|
| Squalane | 0.65 mass% |

The mixture was heated to 80°C, the uniformly dissolved phase A was cooled to 35°C, added to the phase B with stirring, and stirred at 35°C for 10 minutes to precipitate fine needle-like crystals of taurine. A mixture of the phase C was added to the obtained mixture, and the mixture was stirred with a homomixer for 5 minutes and uniformly mixed. Then, the phase D was added, and the mixture was stirred with a homomixer for 10 minutes and emulsified to obtain an emulsion composition.

The shape of the fine crystals of taurine in the obtained composition was needle-like, with a maximum thickness of 30 micrometers and a maximum of 250 micrometers. The use feeling of the obtained composition was smooth, and a composition having good use feeling was obtained.

### W/O type Cream

[A]

| | |
|---|---|
| Purified water | 34.30 mass% |
| Taurine | 7.00 mass% |
| Glycerin | 5.00 mass% |

[B]

| | |
|---|---|
| 1,3-propanediol | 9.00 mass% |
| 1,2-hexanediol | 0.70 mass% |
| Phenoxyethanol | 0.30 mass% |

[C]

| | |
|---|---|
| Isopropyl myristate | 33.70 mass% |
| Jojoba oil | 5.00 mass% |
| Polyglyceryl polyricinoleate-6 | 3.25 mass% |
| Polyglyceryl isostearate-2 | 1.00 mass% |
| Disteardimonium hectorite | 0.75 mass% |

The mixture was heated to 80°C, the uniformly dissolved phase A was cooled to 35°C, added to the phase B with stirring, and stirred at 35°C for 10 minutes to precipitate fine needle-like crystals of taurine. This mixed liquid was added to the homogeneously mixed phase C over 5 minutes while applying a homomixer, and then stirred and emulsified with the homomixer for 10 minutes to obtain a W/O type cream composition.

The shape of the fine crystals of taurine in the obtained composition was needle-like, with a maximum thickness of 25 micrometers and a maximum length of 200 micrometers. The use feeling of the obtained composition was smooth, and a composition having good use feeling was obtained.

### Cosmetic oils

[A]

| | |
|---|---|
| Cetyl 2-ethylhexanoate | 63.40 mass% |
| Hydrogenated polydecene | 29.00 mass% |
| Hydrogenated (styrene/isoprene) copolymer | 7.00 mass% |
| Tocopherol | 0.10 mass% |

[B]

| | |
|---|---|
| Taurine fine needle-like crystals obtained in Example 5 | 0.50 mass% |

The phase A was warmed to 100°C and stirred until homogeneous. The mixture was cooled to 35°C, the phase B was added thereto, and the mixture was stirred and uniformly dispersed to obtain a cosmetic oil composition.

The shape of the fine crystals of taurine in the obtained composition was needle-like, with a maximum thickness of 55 micrometers and a maximum length of 800 micrometers. The use feeling of the obtained composition was smooth, and a composition having good use feeling was obtained.

FIGS. 1 and 2 show the needle-like crystal (Example) and the massive crystal (Comparative Example) as representative examples.

## Claims

1. A skin external agent composition comprising fine needle-like or columnar crystals of taurine having a thickness of less than 150 micrometers and a length of less than 3000 micrometers.

2. The skin external agent composition according to claim 1, further comprising a useful substance.

3. The skin external agent composition according to claim 1 or 2, wherein the fine needle-like or columnar crystals of taurine are grown in an environment in which an aqueous solution in which taurine is dissolved is mixed with or brought into contact with a liquid substance having low solubility of taurine.

4. The skin external agent composition according to claim 3, wherein the liquid substance having low solubility of taurine is a water-soluble organic solvent.

5. A method for producing a fine needle-like or columnar crystals of taurine, the method comprising a step of growing crystals in an environment in which an aqueous solution in which taurine is dissolved is mixed with or brought into contact with a liquid substance having low solubility of taurine.

6. The method according to claim 5, wherein a taurine concentration in the aqueous solution is 3 to 28 mass%.

7. The method according to claim 5 or 6, wherein an amount of the liquid substance having low solubility of taurine is 50 mass% or less with respect to a total amount of the aqueous solution in which taurine is dissolved.

8. The method according to any one of claims 5 to 7, wherein the liquid substance having low solubility of taurine is a water-soluble organic solvent having a taurine solubility of 1.0 g or less per 100 g of the liquid substance (25°C) .

9. The method according to claim 8, wherein the water-soluble organic solvent is one kind or two or more kinds selected from the group consisting of ethanol, isopropyl alcohol, ethylene glycol, 1,2-propanediol, 1,3-propanediol, 1,2-butanediol, 1,3-butanediol, isoprene glycol, diethylene glycol, dipropylene glycol, diethylene glycol monoethyl ether, glycerin, diglycerin, triglycerin, 1,2-pentanediol, 1,2-hexanediol, cyclohexylglycerin, and n-hexylglycerin.

10. A method for producing a skin external agent composition containing fine needle-like or columnar crystals of taurine, the method comprising the steps of:
growing crystals in an environment in which an aqueous solution in which taurine is dissolved is mixed with or brought into contact with a liquid substance having low solubility of taurine; and
blending the fine needle-like or columnar crystals of taurine obtained in the step of growing crystals into the skin external agent composition.

11. The method according to claim 10, wherein a taurine concentration in the aqueous solution is 3 to 28 mass%.

12. The method according to claim 10 or 11, wherein an amount of the liquid substance having low solubility of taurine is 50 mass% or less with respect to a total amount of the aqueous solution in which taurine is dissolved.

13. The method according to claim 10, wherein the liquid substance having low solubility of taurine is a water-soluble organic solvent having a taurine solubility of 1.0 g or less per 100 g of the liquid substance (25°C).

14. The method according to claim 13, wherein the water-soluble organic solvent is one kind or two or more kinds selected from the group consisting of ethanol, isopropyl alcohol, ethylene glycol, 1,2-propanediol, 1,3-propanediol, 1,2-butanediol, 1,3-butanediol, isoprene glycol, diethylene glycol, dipropylene glycol, diethylene glycol monoethyl ether, glycerin, diglycerin, triglycerin, 1,2-pentanediol, 1,2-hexanediol, cyclohexylglycerin, and n-hexylglycerin.

15. The method according to claim 10, wherein the skin external agent composition is a liquid agent, lotion, emulsion, cream, or aqueous gel, and a taurine concentration in an aqueous phase in the blending step is maintained at a saturated concentration or higher, whereby the fine needle-like or columnar crystals of taurine are retained in the skin external agent composition.

16. The method according to claim 10, wherein the skin external agent composition is a cosmetic oil or an ointment, and a product obtained by filtering and drying the fine needle-like or columnar crystals of taurine obtained in the step of growing crystals is blended in the skin external agent composition in the blending step.
